(19) **Europäisches Patentamt**
**European Patent Office**
**Office européen des brevets**

(11) **EP 4 609 820 A1**

(12) **EUROPEAN PATENT APPLICATION**

(43) Date of publication:
**03.09.2025 Bulletin 2025/36**

(21) Application number: **24160208.5**

(22) Date of filing: **28.02.2024**

(51) International Patent Classification (IPC):
**A61B 34/32** *(2016.01)*    **A61B 17/28** *(2006.01)*
**A61B 34/37** *(2016.01)*    **A61F 9/007** *(2006.01)*
**A61B 17/00** *(2006.01)*    **A61B 90/00** *(2016.01)*

(52) Cooperative Patent Classification (CPC):
**A61B 34/32; A61B 34/37; A61F 9/007;**
**A61F 9/00736;** A61B 2017/00115; A61B 2090/061;
A61B 2090/062; A61B 2090/064; A61B 2090/08021;
A61B 2090/3735

(84) Designated Contracting States:
**AL AT BE BG CH CY CZ DE DK EE ES FI FR GB**
**GR HR HU IE IS IT LI LT LU LV MC ME MK MT NL**
**NO PL PT RO RS SE SI SK SM TR**
Designated Extension States:
**BA**
Designated Validation States:
**GE KH MA MD TN**

(71) Applicant: **Carl Zeiss Meditec AG**
**07745 Jena (DE)**

(72) Inventors:
• **BRIEL, Marius**
**76344 Eggenstein-Leopoldshafen (DE)**
• **HAIDE, Ludwig**
**76344 Eggenstein-Leopoldshafen (DE)**
• **REBEKKA, Peter**
**76344 Eggenstein-Leopoldshafen (DE)**
• **HILLENBRAND, Matthias**
**73447 Oberkochen (DE)**

(74) Representative: **DeltaPatents B.V.**
**Fellenoord 370**
**5611 ZL Eindhoven (NL)**

(54) **SURGICAL ROBOTIC SYSTEM AND CONTROL OF SURGICAL ROBOTIC SYSTEM**

(57) A surgical robotic system and method for control of the surgical robotic system are provided. The surgical robotic system is configured to hold a surgical instrument 119 and to provide model-assisted control of the surgical instrument during an intraocular procedure. To establish the model-assisted control of the surgical instrument, the surgical robotic system and method may access model data defining at least two models which each model at least part of the surgical environment, evaluate one or more switching criteria that define conditions for switching between the first model 230 and the second model 240 for the model-assisted control, and when the switching criteria are met, switch between both models in the model-assisted control. By being able to switch between different models, it may not be needed for a single model to be universally applicable in all situations during the intraocular procedure, which may be exceedingly difficult and/or may greatly increase the complexity of a single model beyond the combined complexity of two or more separate models.

Fig. 6

**Description**

**TECHNICAL FIELD**

The invention relates to a surgical robotic system for use in an intraocular procedure. The invention further relates to a method for controlling a surgical robotic system during an intraocular procedure, and to a computer program comprising instructions for causing a processor system to perform the method.

**BACKGROUND**

Intraocular surgical procedures increasingly involve the use of surgical robotic systems. Rather than operating entirely autonomously, such surgical robotic systems are expected for the near future to remain at least in part under the control of a human operator. For example, the human operator may directly or indirectly control the movement of a surgical instrument mounted to a surgical arm of the surgical robotic system. Nevertheless, it is expected that future surgical robotic systems may be more autonomous and require less or even no involvement of a human operator.

A surgical robotic system designed for intraocular procedures may be provided with a surgical arm which comprises a movable arm part, with the movable arm part comprising an end effector for holding a surgical instrument. Accordingly, the surgical instrument may be positioned by the surgical arm. An actuator subsystem may be provided for actuating the movable arm part to control a pose of the surgical instrument. Here, the term 'pose' may refer to a position and orientation of surgical instrument. Through such actuation, the surgical instrument may be moved in a lateral direction, for example relative to a retinal surface, and in a longitudinal direction along a longitudinal axis of the surgical instrument. Longitudinal movement may typically allow the surgical instrument to be moved towards and away from a surgical target within an interior of the eye. Accordingly, the surgical instrument may be used to modify (biological) tissue near the surgical target, to deliver an agent to the surgical target, etc. Examples of surgical instruments include, but are not limited to, forceps, mechanical cutters, coagulation cutters, scissors, injection needles, sealing devices, etc.

Surgical robotic systems of the above type are known per se. For example, US10350014B2 describes a surgical robotic system for use in a surgical procedure, comprising a surgical arm comprising a movable arm part, the movable arm part comprising an instrument connector for mounting of a surgical instrument, the surgical instrument having a longitudinal axis, the movable arm part having at least one degree-of-freedom to enable longitudinal movement of the surgical instrument along the longitudinal axis of the surgical instrument towards a surgical target. The surgical robotic system is said to further comprise a human machine interface for receiving positioning commands from a human operator for controlling the longitudinal movement of the surgical instrument, an actuator configured for actuating the movable arm part to effect the longitudinal movement of the surgical instrument, and a processor configured for controlling the actuator in accordance with the positioning commands.

While the use of surgical robotic systems of the above type in intraocular procedures offers numerous benefits, including enhanced precision and dexterity, an issue is the potential for the human operator to unintentionally inflict damage within the eye due to the human operator incorrectly controlling the surgical instrument's pose. The problem of incorrect control may be aggravated in cases where the human operator is granted extensive control over the pose of the surgical instrument. Namely, such extensive control may be challenging for the human operator to manage, particularly during moments of intense focus on executing specific surgical actions.

It is known for a surgical robotic system to use a model which models the surgical environment to establish model-assisted control of the surgical instrument. Such model-assisted control may for example be used to complement sensing, since intraoperative sensing may be very noisy, to enable automation, etc. For example, the aforementioned US10350014B2 describes the use of an algebraic geometry or a numerical model to construct a virtual bound. The virtual bound may be used by the surgical robotic system to prevent the human operator from moving the surgical instrument past the virtual bound to, for example, protect delicate tissue.

**SUMMARY**

It would be desirable to further improve upon such model-assisted control.

In accordance with a first aspect of the invention, a surgical robotic system is provided for use in an intraocular procedure, comprising:

- an end effector for holding a surgical instrument;
- an actuator subsystem;
- a sensor interface to a sensor configured to capture sensor data of at least part of a surgical environment during the intraocular procedure;
- a processor subsystem configured to:

- control the actuator subsystem to control a pose of the surgical instrument during the intraocular procedure; and
- in the control, use a model which models the surgical environment to establish a model-assisted control of the surgical instrument;

wherein the processor subsystem is further configured to:

- access at least a first model and a second model, wherein each model is configured to model at least part of the surgical environment;

and during the intraocular procedure:

- access the sensor data captured by the sensor;
- adapt at least one of said models to the sensor data to update said model to better model a currently captured part of the surgical environment;
- assess one or more switching criteria that define conditions for switching between the first model and the second model for the model-assisted control; and
- when the one or more switching criteria are met, switch between the first model and the second model for the model-assisted control.

In accordance with a further aspect of the invention, a computer-implemented method is provided for controlling a surgical robotic system during an intraocular procedure, wherein the surgical robotic system comprises:

- an end effector for holding a surgical instrument;
- an actuator subsystem;
- a sensor interface to a sensor configured to capture sensor data of at least part of a surgical environment during the intraocular procedure;

wherein the method comprises:

- controlling the actuator subsystem to control a pose of the surgical instrument during the intraocular procedure;
- in the control, using a model which models the surgical environment to establish a model-assisted control of the surgical instrument;

wherein the method further comprises:

- accessing at least a first model and a second model, wherein each model is configured to model at least part of the surgical environment;

and during the intraocular procedure:

- accessing the sensor data captured by the sensor;
- adapting at least one of said models to the sensor data to update said model to better model a currently captured part of the surgical environment;
- assessing one or more switching criteria that define conditions for switching between the first model and the second model for the model-assisted control; and
- when the one or more switching criteria are met, switching between the first model and the second model for the model-assisted control.

In accordance with a further aspect of the invention, a transitory or non-transitory computer-readable medium is provided comprising data representing a computer program, the computer program comprising instructions for causing a processor system to perform the above-identified computer-implemented method.

The presently disclosed measures provide a surgical robotic system which comprises an end effector for holding a surgical instrument. For example, the end effector may comprise an instrument connector for mounting the surgical instrument, for example in a removable manner. The surgical robotic system may further comprise an actuator subsystem for controlling the pose of the surgical instrument during an intraocular procedure. Another term for actuator subsystem is driving mechanism. The adjustment of the pose may involve adjusting the position and/or orientation of the surgical instrument, for example to advance the surgical instrument towards and/or retract away from a surgical target, or to move the surgical instrument laterally along a plane perpendicular to a longitudinal axis of the surgical instrument. In a specific example, the surgical robotic system may comprise a surgical arm, the surgical arm may comprise a movable arm part, and the movable arm part may comprise the end effector. In such a specific example, the pose of the surgical instrument may be adjusted by the actuator subsystem adjusting the pose of the moveable arm part.

A processor subsystem may be provided to control the actuator subsystem to control the pose of the surgical instrument during the intraocular procedure. Such control may directly or indirectly involve a human operator. For example, the surgical robotic system may comprise a human machine interface for enabling a human operator to provide operator commands to the surgical robotic system, and the processor subsystem may be configured to control the actuator subsystem to control the pose of the surgical instrument based on the operator commands. Such type of control may also be referred to as master-slave control. When using master-slave control, the human operator may exert at least partial control over the pose of the surgical instrument. In other examples, the processor subsystem may be configured to autonomously or semi-autonomously control the pose of the surgical instrument.

It is noted that surgical robotic systems having the functionality described in the two preceding paragraphs may be known per se from the field of medical robotics.

In accordance with the presently disclosed measures, the processor subsystem is further configured to use a model which models the surgical environment to establish model-assisted control of the surgical instrument. For example, the model may be a geometric model of the surgical environment, e.g., of an interior of the eye, or a biomechanical model of a surgical target in the surgical environment, e.g., of a tissue layer. In this respect, it is noted the model may not need to model all of the surgical environment, e.g., in terms of detail and/or extent. The model may thus be an incomplete model of the surgical environment, e.g., an at least partial model. For example, the model may model one or more anatomical structures in the surgical environment and thereby model part of the surgical environment. The model may be used by the processor subsystem for the aforementioned model-assisted control. Such model-assisted control may take various forms, including forms which may be known per se. For example, the processor subsystem may use the model to establish a virtual bound for the surgical instrument to protect delicate tissue. It will be appreciated, however, that the model-based assistance provided by a surgical robotic system may go beyond the protection of delicate tissue, for example allowing the surgical robotic system to automatically perform certain actions to support the human operator.

In accordance with the presently disclosed measures, the processor subsystem may be provided with at least two models each modelling at least part of the surgical environment. The part of the surgical environment modelled by the respective models may be a same part, or different yet partially overlapping parts, or different nonoverlapping parts, etc. The surgical robotic system may further comprise a sensor interface to a sensor. The sensor may be configured to capture sensor data of at least part of a surgical environment during the intraocular procedure. For example, the sensor may be an intraoperative OCT probe configured to acquire sensor data during the intraocular procedure. The processor subsystem may be configured to access the sensor data during the intraocular procedure, for example in a periodic or generally repeated manner, and may adapt at least one of the models to the sensor data. Such sensor data acquisition and model adaptation may be performed repeatedly during the intraocular procedure, for example in response to newly captured sensor data or when the sensor data pertains to a different part of the surgical environment. By way of such model adaptation, the model may be updated to better model the currently captured part of the surgical environment. It will be appreciated that by updating the model in the above manner, also non-captured parts of the surgical environment may be better modelled, e.g., by the model generally better fitting the surgical environment at hand. Such better modelling may for example comprise the deviation between an actual geometry of the surgical environment and a modelled geometry being reduced. This way, the model may more accurately model the surgical environment at hand.

The presently disclosed measures further involve the processor subsystem being configured to switch between a first model and a second model for the model-assisted control. Such switching may be explained as follows. The model-assisted control may use a model to provide the control assistance. For example, the model may be used for a specific purpose, for example to establish a virtual bound. The processor subsystem may be configured to switch between both models. As a result, at some time instances the virtual bound may be established based on the first model while at other time instances the virtual bound may be established based on the second model. It will be appreciated that the switching does not preclude the other model, which may not be used for this specific purpose at a given time instance, from being concurrently used for another purpose in the model-assisted control. In other words, 'switching between models' may refer to a switching between the models with respect to a specific use in the model-assisted control and may not preclude the other model being also used in the model-assisted control but for a different use or purpose.

The processor subsystem may be configured to assess when to switch between the models. For that purpose, one or more switching criteria may be provided which define conditions for switching between the models. The processor subsystem may assess the switching criteria to determine when the conditions to switch are satisfied, and if this is the case, the processor subsystem may effect the switch between the models. For example, the switching criteria may be assessed periodically or generally repeatedly. It is noted that the switching criteria may define conditions when to switch between models, e.g., from a first model to a second model, but may in some examples also define conditions when to switch back, e.g., from the second model to the first model. This way, the processor subsystem may switch back and forth between the models when the respective switching conditions are met.

By way of the presently disclosed measures, the system may alternate between at least two models in the model-assisted control. These measures may be based at least in part on the insight that a single model may not be universally applicable, for example in its ability to accurately model the surgical environment at all times. For example, in regard to a model which is adaptive to sensor data, the inventors have recognized that such adaptative models may in some situations be effective in modelling the surgical environment but in other situations ineffective, e.g., when insufficient sensor data is available, e.g., in terms of amount or coverage of the surgical environment, when the sensor data is noisy, etc. By having the possibility to switch between models, the weakness of such adaptive models may be mitigated by the processor subsystem being able to switch to a non-adaptive model or to a simpler yet more stable adaptive model. Likewise, the advantages of such adaptive models may be exploited by the processor subsystem being able to switch to an adaptive model which more accurately models the surgical environment than a presently used non-adaptive model or simpler adaptive model. By providing such switching functionality, the advantages of two or more models may be combined dynamically during the procedure. Generally, both models may be complementary in some respects or at least have different strengths and/or weaknesses. The switching may therefore exploit the respective strengths and mitigate the respective weakness of the models. It is therefore not needed for a single model to be universally applicable in all situations during the intraocular procedure, which may be exceedingly difficult and/or may greatly increase the complexity of a single model beyond the combined complexity of two separate models. In addition, the switching criteria may allow the processor subsystem to autonomously determine when to switch, thus relieving the human operator of the burden to manually evaluate the switching criteria and issue the switch command.

The following optional aspects are described with reference to the surgical robotic system but may equally apply to the computer-implemented method for controlling the surgical robotic system and to the corresponding computer program.

Optionally, the first model and the second model differ by at least one characteristic, wherein the processor subsystem is configured to access data which at least partially characterizes a current state of the surgical robotic system and/or the intraocular procedure, and wherein the one or more switching criteria are defined to determine a utility and/or suitability of a respective model in the current state by evaluating the utility and/or suitability of the characteristic of the respective model in the current state. Both models may differ in one or more characteristics, and thereby, both models may have different distinguishing qualities. These difference(s) may affect the suitability of a model in a certain situation. For example, if one of the models is detailed yet potentially less stable, for example by being sensitive to noisy sensor data, such a model may not be deemed suitable to be used in situations where the sensor data is likely to be noisy. The processor subsystem may be configured to characterize the current situation by determining, at least in part, a current state of the surgical robotic system and/or a current state the intraocular procedure. For example, the processor subsystem may assess one or more operational parameters of the surgical robotic system to determine the current state of the surgical robotic system. Another example is that the processor subsystem may access surgical planning data and may, based on the surgical planning data, determine a current phase of the intraoperative procedure, thereby characterizing the current state of the intraocular procedure. Having characterized the aforementioned state(s), the processor subsystem may determine the suitability and/or utility of a respective model for the present situation based on at least the characteristic(s) for which both models differ. In other words, the processor subsystem may at least take one or more distinguishing qualities of the respective models into account to determine their suitability and/or utility for the present situation. Here, 'suitability' may refer to an assessment of whether a model is fit for use at all, e.g., whether a minimum degree of suitability is reached, whereas 'utility' may quantify a degree of suitability above said minimum. The switching criteria may thus take the suitability and/or utility into account, which may allow the processor subsystem to switch to a respective model when the respective model is deemed to be suitable, for example most suitable of all models or at least suitable to a (pre)defined degree.

Optionally, the one or more switching criteria are defined to compare the utility and/or suitability of both models to determine when to switch between the first model and the second model, or defined to compare the utility and/or suitability of a respective model to a reference. By directly comparing the utility and/or suitability of both models, the processor subsystem is enabled to switch to a respective model which is deemed to be most suitable in the current situation. By comparing the utility and/or suitability to a reference, the processor subsystem is enabled to switch to a respective model when it is deemed to have met certain (minimum) requirements.

Optionally, the one or more switching criteria are defined to switch from the first model to the second model:

- when a scope of the first model is reached;
- when the second model is deemed sufficiently reliable, for example based a confidence level outputted by the second model or based on a comparison between a modelled quantity by the second model and an observed quantity from the sensor data or based on a defined amount of sensor data having been used to adapt the second model or based on a temporal stability of a modelling by second model;
- when a defined distance to an anatomical structure is reached, with the second model providing a different level of detail of the anatomical structure;
- when a defined movement speed of the surgical instrument is exceeded, with the second model being more computationally efficient to update and/or evaluate than the first model;

- when a defined surgical phase is reached, with the second model being configured to be used in the defined surgical phase;
- when the sensor data is unavailable or unreliable, with the second model being a fallback model for such unavailability or unreliability of the sensor data.

The above are non-limiting examples of conditions which may be evaluated by the processor subsystem and which may trigger a switch between the models.

Optionally, the first model and the second model differ in terms of complexity, for example in terms of their capability to model the surgical environment or in terms of a computational complexity of updating and/or evaluating said models. The inventors have considered that it may be desirable to provide a more complex model and a less complex model. More complex models may be more adapt at modelling the surgical environment, e.g., by being able to model the surgical environment more accurately, by capturing more details, by modelling a larger part of the surgical environment, etc. However, more complex models may not always be universally applicable during the intraocular procedure. For example, complex models may, in the case of adaptive models, require more sensor data to accurately model the surgical environment, may be more sensitive to noise in the sensor data, may require more computational resources to evaluate, may require more computational resources to update, etc. As such, there may be situations in which the use of a more complex model is not preferred, and rather, a simpler model may be preferred. Such simpler models may be simpler in terms of requiring less or no sensor data to model the surgical environment, being less sensitive to noise in the sensor data, requiring fewer computational resources to evaluate, requiring fewer more computational resources to update, etc. The processor subsystem may evaluate the switching criteria to determine if the more complex model may be used, for example by evaluating the strength and weaknesses of such a more complex model in view of the present situation, and may otherwise revert to the simpler model, for example when the weaknesses of the more complex model are deemed to outweigh the strengths of the more complex model.

Optionally, the first model and the second model differ in terms of:

- a number of parameters which define a respective model;
- an amount of sensor data which is needed to obtain a satisfactory accurate modelling of the surgical environment;
- a level of detail which a respective model is capable of modelling;
- an extent of the surgical environment which a respective model is capable of modelling;
- a computational complexity of evaluating a respective model;
- a computational complexity of updating a respective model to new sensor data;
- an average level of confidence of predictions provided by a respective model;
- a type of model, for example a machine learning model and a non-machine learning model or different types of machine learning models.

The above may be non-limiting examples of differences between the models which may result in the models differing in terms of complexity.

Optionally, one or each respective model is a geometric model and/or a biomechanical model of the surgical environment.

Optionally, the processor subsystem is configured to adapt both the first model and the second model based on sensor data captured during the intraocular procedure. In some examples, both models may be adaptive models, in that both models may be adapted to the surgical environment by adaptation to sensor data.

Optionally, the sensor interface is configured to, during the intraocular procedure, access first sensor data captured by a first sensor and second sensor data captured by a second sensor, wherein the processor subsystem is configured to adapt the first model based on the first sensor data and the second model based on the second sensor data. In some examples, the respective models may be adapted based on different types of sensor data. For example, one model may be adapted based on intraocular sensor data while another model may be adapted based on extraocular sensor data. Another example is that one model may be adapted based on sensor data from one sensor modality, e.g., OCT, while another model may be adapted based on sensor data from another sensor modality, e.g., ultrasonic or time-of-flight or image data. Another example is that one model may be adapted based on intraoperative sensor data while another model may be adapted based on preoperative sensor data. By being adapted(-able) to different types of sensor data, both models may have different strengths and weaknesses. By switching between both models, the respective strengths of the models may be exploited and their respective weaknesses mitigated. Optionally, one or each respective model is:

- a parameterized model, wherein the parameterized model is adapted to the sensor data by adapting one or more parameters of the parameterized model; or
- a machine learning model, wherein the machine learning model is adapted to the sensor data by continuous, incremental, or online learning.

An example of an adaptive model is a parameterized model of which parameters may be adjusted so that an output of the parameterized model better fits the available sensor data. Another example of an adaptive model is a machine learning model which may be adapted by way of continuous learning, or by way of incremental learning, or by way of online learning, for example by adjusting its weights, so that a modelling by the machine learning model better fits the available sensor data.

Optionally, the first model is a global model of the eye and the second model is a local model of a part of the eye.

Optionally, the first model is a spherical model of the eye and wherein the second model is an ellipsoid model of the eye. A spherical model may be simpler yet more robust to fit to the surgical environment using sensor data. An ellipsoid model may better capture the geometry of the eye but may be less robust to fit to the surgical environment using sensor data, for example by requiring more sensor data. By switching between both models, the respective strengths of the models may be exploited and their respective weaknesses mitigated. For example, the spherical model may be used as a default model until it is deemed that the ellipsoid model is sufficiently robust, e.g., by having been adapted to a sufficient amount of sensor data.

Optionally, the sensor comprises an OCT probe, for example integrated into or attached to the surgical instrument.

Optionally, the processor subsystem is configured to use a respective model in the control of the actuator subsystem to:

- limit an adjustment of the pose of the surgical instrument by a human operator of the surgical robotic system;
- adjust the pose of the surgical instrument;
- perform an action with the surgical instrument;
- adjust an operating parameter of the surgical instrument; and/or
- provide sensory-perceptible feedback to the human operator.

The above examples represent different ways in which a surgical robotic system may use a model to assist in the control of the surgical instrument, or in other words, different types of model-assistance. For example, the surgical robotic system may use a model to impose limitations on the pose of the surgical instrument, e.g., for protection of delicate tissue or to simplify the control for the human operator. Examples of such limitations include, but are not limited to, reducing a range and/or direction of movement or rotation, disallowing movement or rotation in a certain direction or along a certain axis, limiting the acceleration, etc. Other examples of model-assistance include the surgical robotic system automatically adjusting the pose of the surgical instrument, performing an action with the surgical instrument, and/or adjusting an operating parameter of the surgical instrument, for example to temporarily or continuously assist the human operator in the control of the surgical instrument or to assist the surgical robotic system in autonomously carrying out the intraocular procedure. An example of an automatic adjustment of the pose of the surgical instrument is the retraction of the surgical instrument, e.g., away from an anatomical structure. Such a retraction may for example be triggered automatically based on an evaluation of either or both models, for example if both models are in significant disagreement with each other. Another example is that the model may be used to provide feedback to the human operator which may allow the human operator to better control the surgical instrument.

It will be appreciated by those skilled in the art that two or more of the above-mentioned embodiments, implementations, and/or aspects of the invention may be combined in any way deemed useful.

Modifications and variations of the computer-implemented method and/or the computer-readable medium, which correspond to the described modifications, variations, and optional aspects of the surgical robotic system, may be carried out by a person skilled in the art on the basis of the present description.

## BRIEF DESCRIPTION OF THE DRAWINGS

These and other aspects of the invention are apparent from and will be elucidated with reference to the embodiments described hereinafter. In the drawings,

Fig. 1 shows a schematic representation of a surgical robotic system;
Fig. 2 shows a surgical instrument passing through a trocar during minimally invasive surgery, the surgical instrument having four degrees of freedom (DoFs);
Fig. 3 shows a joint diagram illustrating the kinematics of a movable arm part of a surgical arm for use in minimally invasive surgery;
Fig. 4 shows a joint diagram illustrating the kinematics of a motion controller;
Fig. 5 shows a surgical instrument in an eye according to an embodiment of the invention;

Fig. 6 illustrate a spherical model and an ellipsoid model which are each configured to model an interior of the eye;

Fig. 7 shows examples of utility functions for two models, each utility function expressing a utility of a respective model as a function of time;

Fig. 8 schematically shows a method for controlling a surgical robotic system during a surgical procedure; and

Fig. 9 shows a non-transitory computer-readable medium;

It should be noted that items which have the same reference numbers in different figures, have the same structural features and the same functions, or are the same signals. Where the function and/or structure of such an item has been explained, there is no necessity for repeated explanation thereof in the detailed description.

**List of reference numerals**

The following list of references and abbreviations is provided for facilitating the interpretation of the drawings and shall not be construed as limiting the claims.

| | |
|---|---|
| 20 | user interface subsystem |
| 22 | user inputs |
| 30 | sensor |
| 32 | sensor data |
| 40 | processor subsystem |
| 42 | actuation commands |
| 60 | actuator subsystem |
| 62 | actuation of surgical arm |
| 80 | surgical arm |
| 82 | movable arm part |
| 100 | surgical robotic system |
| 104 | $\vec{e}_x$, axis of coordinate system fixed to the instrument tip, orthogonal to the instrument longitudinal axis |
| 105 | $\vec{e}_y$, axis of coordinate system fixed to the instrument tip, orthogonal to the instrument longitudinal axis |
| 106 | $\vec{e}_z$, axis of a cartesian coordinate system, aligned with the instrument longitudinal axis |
| 107 | $\phi$, rotation of surgical instrument, laterally displacing its tip |
| 108 | $\psi$, rotation of surgical instrument, laterally displacing its tip |
| 109 | $z$, longitudinal (along its longitudinal axis) translation of surgical instrument, or penetration direction, or advancing direction |
| 110 | $\theta$, rotation of surgical instrument around its longitudinal axis |
| 111 | $\varphi$, rotational DoF of a movable arm part |
| 112 | $\psi$, rotational DoF of a movable arm part |
| 113 | $z$, translational DoF of a movable arm part |
| 114 | $\theta$, rotational DoF of a movable arm part |
| 115 | $\varphi_m$, rotational DoF of motion controller |
| 116 | $\psi_m$, rotational DoF of motion controller |
| 117 | $z_m$, translational DoF of motion controller |
| 118 | $\theta_m$, rotational DoF of motion controller |
| 119 | surgical instrument |
| 121 | sensor or sensor part |
| 122 | surgical instrument tip |
| 123 | surgical target |
| 124 | trocar |
| 125 | remote centre of motion (RCM) |
| 126 | button on motion controller gripper |
| 127 | optical beam |
| 200 | eye |
| 210 | retinal |
| 220 | distance measurement |
| 230 | spherical model of interior of eye |
| 240 | ellipsoid model of interior of eye |
| 300 | time axis |
| 310 | utility axis |
| 320 | utility function of first model |

330    utility function of second model
340    switching point
400    method of controlling a surgical robotic system
410    accessing first model and second model
420    steps performed during surgical procedure
430    accessing sensor data captured by sensor
440    adapting at least one model to the sensor data
450    assessing switching criteria to switch between models
460    if switching criteria are met, switching between models
500    non-transitory computer readable medium
510    data representing computer program

## DESCRIPTION OF EMBODIMENTS

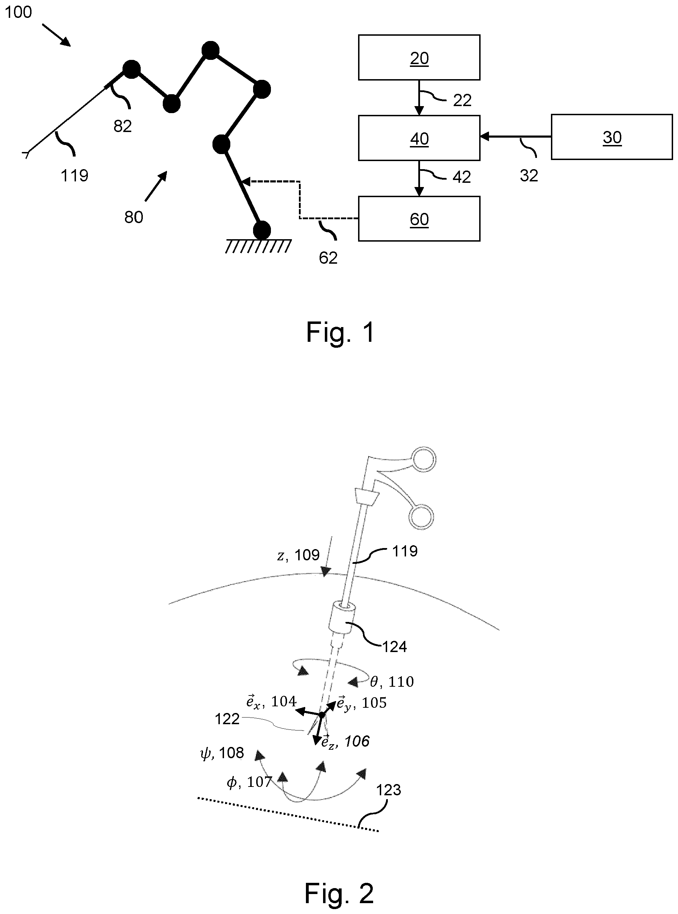

The following embodiments relate to a surgical robotic system for use in a surgical procedure. During the surgical procedure, the surgical robotic system may control a pose of a surgical instrument, and in the control, use a model which models the surgical environment to establish model-assisted control of the surgical instrument. The following describes such model-assisted control within the context of a specific surgical robotic system, namely a surgical robotic system which is configured for intraocular surgery and described with reference to, inter alia, Figs. 1-6. Nonetheless, the embodiments described in this specification are not confined solely to this type of surgical robotic system, nor to the field of intraocular surgery. Rather, the embodiments can be adapted and applied to other types of surgical robotic systems for use in intraocular procedures, as well as to other types of surgical robotic systems for use in other types of surgical procedures, such as microsurgical procedures, minimally invasive procedures, endoluminal procedures, etc. Any references to intraocular surgery and intraocular structures are therefore to be understood as applying also to other types of surgical procedures and corresponding types of anatomical structures.

**Fig. 1** schematically shows a surgical robotic system 100 for use in an intraocular surgical procedure. The surgical robotic system 100 may comprise a surgical arm 80. The surgical arm 80 may comprise a movable arm part 82 which may comprise an end effector (not explicitly shown in Fig. 1) for holding a surgical instrument 119. For example, the surgical instrument 119 may be an irrigation/aspiration instrument for irrigating or aspirating fluid at a desired target location, a photocoagulation instrument for applying optical energy at a desired distance from a target, a vitrectomy instrument for cutting and aspirating the vitreous humor or other fluidics at a desired distance from a target, a tissue manipulating instrument, which may need to be placed at the interface of a tissue, or the like. To hold the surgical instrument 119, the end effector may comprise an instrument connector to which the surgical instrument 119 may be mounted. The movable arm part 82 may have at least three degrees-of-freedom (DoF) to enable longitudinal movement of the surgical instrument towards a surgical target within the eye and lateral movement of the surgical instrument in a plane normal to said longitudinal movement. Here, longitudinal movement may refer to a movement of the surgical instrument 119 along its longitudinal axis (not separately shown in Fig. 1). In some embodiment, the surgical robotic system 100 may be configured to enable lateral movement in a curved plane. The curvature of the curved plane may for example follow a curvature of the interior of the eye. Such type of lateral movement along a curved plane may be obtained by the surgical robotic system adjusting the longitudinal position of the surgical instrument 119 during lateral movement. The shape of the curved plane may for example be determined based on a model of the eye or of an intraocular structure, e.g., the retina. The model-based adjustment of the position of the surgical instrument 119 is an example of model-assisted control as referenced elsewhere in this specification.

In some embodiments, the surgical robotic system 100 may further comprise a user interface subsystem 20 for receiving user inputs 22 from a user. The user interface subsystem 20 may thus represent at least a user *input* interface, and in some embodiments, as also discussed elsewhere, additionally or alternatively a user *output* interface. The user inputs 22 may for example comprise positioning instructions from a human operator, e.g., a surgeon, to enable the human operator to determine a trajectory of the surgical instrument. In some embodiments, the positioning instructions may be positioning commands for directly controlling the movement of the surgical instrument. In other embodiments, the positioning instructions may be provided as input during a planning procedure in which the trajectory of the surgical instrument is planned. Other types of user inputs may for example include confirmation inputs indicating instructions to continue with a procedure or procedural step, and/or input information, such as indicating an operating parameter and/or an indication of a surgical target position or the like. Examples of user input interfaces for receiving user inputs from a user include, but are not limited to, a keyboard, a mouse, a touch-sensitive surface, a joystick, a foot pedal, a microphone, a gesture recognition system, etc. The user input interface may employ any suitable input modality, such as touch, push-actions, voice commands, eye movements, gesture recognition, etc.

The surgical robotic system 100 may further comprise an actuator subsystem 60 configured and arranged for actuating the movable arm part to effect the longitudinal movement and lateral movement of the surgical instrument. The actuator subsystem 60 may comprise any suitable actuator(s), e.g., from the field of surgical robots, or from the more general field of actuators. In particular, the actuator subsystem 60 may comprise a plurality of actuators which together provide the actuation of the movable arm part 60 along the three or more DoFs. Accordingly, it will be appreciated that any reference to a specific configuration of the actuator subsystem 60 may be understood as referring to a (joint) configuration of such a plurality of actuators.

Fig. 1 shows the actuation of surgical arm 80 schematically, namely as a dashed line 62. It is noted that, although shown separately of the surgical arm 80, the actuator subsystem 60 may be integrated into, or mounted to, the surgical arm 80.

The surgical robotic system 100 may further comprise a sensor interface to a sensor 30 to access sensor data 32 acquired or generated by the sensor 30. For example, the sensor 30 may be or comprise an optical coherence tomography (OCT) probe. The OCT probe may for example be an optical fibre which is attached to or integrated in the surgical instrument 119, with the optical fibre being connected to a remotely located OCT sensor. In other examples, the OCT probe may comprise an OCT sensor which is directly attached to or directly integrated in the surgical instrument 119. The sensor data 32 provided by the OCT probe may comprise A-scans, which may comprise line measurements and defined as an intensity as a function of the distance, B-scans forming a 2D image, C-scans, e.g., from multiple B-scans, or the like. It is noted that even though the sensor 30 is shown in Fig. 1 to be separate from the surgical instrument 119, the sensor or part of the sensor may be attached to or integrated in the surgical instrument 119. In other examples, the sensor 30 may, in addition or alternatively to the OCT probe, comprise an external OCT sensor, a stereo camera arranged for image capture through a microscope, an optical interferometric sensor integrated in or attached to the surgical instrument, a time-of-flight sensor integrated in or attached to the surgical instrument, an ultrasonic sensor integrated in or attached to the surgical instrument, etc. In general, the sensor 30 may be an intraoperative sensor, such as an intraocular sensor, e.g., insertable into the eye, or an external sensor. In some embodiments, the surgical robotic system 100 may comprise a microscope which is communicatively coupled to the surgical robotic system 100.

The surgical robotic system 100 may further comprise a processor subsystem 40 configured for controlling the actuator subsystem 60 to control a pose of the surgical instrument 119 during the intraocular procedure. For example, in an epiretinal membrane peeling procedure, the pose of the surgical instrument 119 may be controlled to grasp and peel the epiretinal membrane starting from an edge of the epiretinal membrane. Another example is cataract surgery, during which the pose of the surgical instrument 119 may be controlled in relation to the lens or the capsular bag. Another example is glaucoma surgery, during which the pose of the surgical instrument 119 may be controlled in relation to the trabecular meshwork.

As also discussed elsewhere, the processor subsystem 40 may function in an operator control mode in which the processor subsystem 40 directly carries out positioning commands received from a human operator, or in an autonomous control mode in which the processor subsystem 40 autonomously controls the position of the surgical instrument, e.g., according to a pre-planned trajectory and sensor data, or in a semi-autonomous control mode which combines aspects of the operator control mode and the autonomous control mode. In particular, the processor subsystem 40 may be configured to only function in one control mode, or may be configured to switch between all or a subset of these control modes, for example at the request of a human operator. During the intraocular procedure, and thus during, or time-alternatingly with, the control of the actuator subsystem 60, the processor subsystem 40 may use a model of the surgical environment to assist in the control of the surgical instrument. For example, the model may be a geometric model of the surgical environment. Such model-based assistance may be provided in the operator control mode or in the semi-autonomous control mode to assist the human operator. For example, the processor subsystem 40 may use the model to limit an adjustment of the pose of the surgical instrument by the human operator, to autonomously adjust the pose of the surgical instrument, to autonomously perform an action with the surgical instrument, to autonomously adjust an operating parameter of the surgical instrument, to provide sensory-perceptible feedback to the human operator, etc. Additionally, or alternatively, the model-based assistance may be provided during the autonomous control mode to assist the surgical robotic system in carrying out surgical or navigational tasks.

In general, the processor subsystem 40 may use the model to take actions during the surgical procedure. In particular, an action may be triggered if the modelling of the surgical environment by the model indicates that criteria for taking the action are met. These criteria may be predefined. An example of an action is adjusting the control of the actuator subsystem, for example to slow down or stop a movement of the surgical instrument or to execute a surgical action. Another example of an action is an emergency action, such as an emergency retraction of the surgical instrument. Another example of an action may be the control of the surgical instrument itself, for example to perform a surgical action. Another example of an action may be the control of a further surgical instrument held by the surgical robotic system. Yet another example of an action may be the generation and outputting of a sensory perceptible feedback signal to a user. The sensory perceptible feedback signal may for example be an audible signal, a haptic signal, and/or a visual signal. For example, the processor subsystem 40 may be configured to output the sensory perceptible feedback signal via the user interface subsystem 20. In such an example, the user interface subsystem 20 may thus at least represent an output interface. For example, the sensory perceptible feedback signal may be rendered visually, e.g., as an overlay over an image of the surgical field, as an audio output, e.g., via a speaker of the user interface subsystem 20, and/or as a visual numerical output, e.g., on a display of the user interface subsystem 20. Another example is that a haptic signal may be provided via the user input interface, e.g., by providing a haptic signal via a motion controller held by the human operator.

**Fig. 2** shows a surgical instrument 119 passing through a trocar 124 during minimally invasive surgery. For example, in case of vitreoretinal surgery, the trocar 124 may be placed in the sclera. Rotating around and translating through the trocar may be possible in four DoF, e.g., the rotations $\phi$ 107, $\psi$ 108, $\theta$ 110 and the translation $z$ 109 to approach or penetrate a surgical target 123. Further shown are a tip 122 of the surgical instrument 119 and three axes 104-106 of a coordinate system fixed to the instrument tip 122, with $\vec{e}_z$ 106 aligned with the longitudinal axis of the surgical instrument 119. Rotations $\phi$ 107 and $\psi$ 108 may result in a lateral displacement of the instrument tip 122, respectively in the direction $\vec{e}_y$ 105 and in the direction $\vec{e}_x$ 104. The translation $z$ 109 may result in a longitudinal movement of the surgical instrument tip 122.

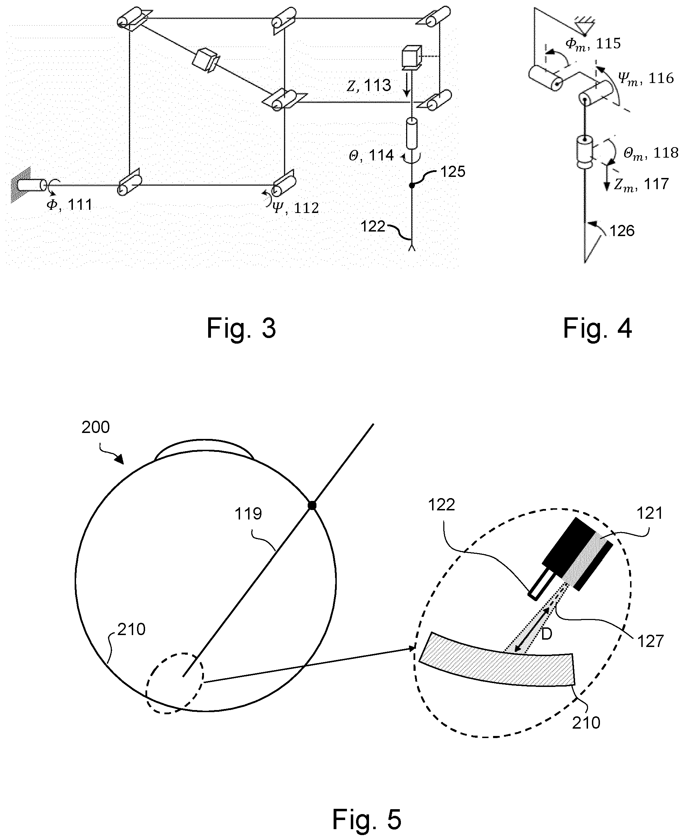

**Fig.** 3 shows a joint diagram illustrating the kinematics of a movable arm part of a surgical arm for use in an intraocular surgical procedure, and in particular a minimally invasive surgery. In the example of Fig. 3, the surgical robotic system comprises a surgical arm, with the surgical arm comprising a movable arm part having DoFs $\Phi$ 111, $\Psi$ 112, $Z$ 113 and $\Theta$ 114, allowing instrument motions 107-110 as previously shown in Fig. 2, resulting in movements of the surgical instrument tip 122. The DoFs may be arranged such that there is a point on the surgical instrument 122 that does not move in space, termed the remote centre of motion (RCM) 125. By moving the base of the surgical arm, the movable arm part may be positioned such that the remote centre of motion 125 of the surgical instrument is positioned at the trocar. Respective actuators may be arranged to effect movement in all four DoFs 111-114.

As previously discussed with reference to Fig. 1, the surgical robotic system may comprise a user input interface for receiving user inputs, such as positioning instructions, from a human operator, e.g., a surgeon or healthcare provider. In some embodiments, the user interface may comprise or be constituted by a motion controller such as a joystick. In some embodiments, the motion controller may be an external device with which the user interface or the processor subsystem 40 is configured to interface. In some embodiments, the motion controller may be comprised in the surgical robotic system as a further component, e.g., configured to interface with the processor subsystem 40 and/or the user interface subsystem 20. **Fig. 4** shows a joint diagram illustrating the kinematics of such a motion controller. Here, the motion controller is shown to have DoFs $\Phi_m$ 115, $\Psi_m$ 116, $Z_m$ 117 and $\Theta_m$ 118. The user may provide user inputs, such as positioning commands to directly control the movement of the movable arm part, for example by holding the motion controller at a gripper part, pressing the button 126 and moving the gripper part of the motion controller in space.

**Fig. 5** shows a surgical instrument 119 in an eye 200. The tip 122 of the surgical instrument 119 is also shown magnified. The tip 122 of the surgical instrument 119 may be an operative tip in that the tip may be operatively involved in the intraocular procedure. As illustrated on the left hand side of Fig. 5, the surgical instrument 119 is shown inserted into the eye 200. The surgical instrument 119 may be approaching the retina 210 as illustrated in this figure, although this is merely exemplary and the invention is not limited thereto. In some embodiments, the surgical instrument 119 may approach another area, structure, or tissue layer of the eye, such as a capsular bag or a part of the eye's drainage system. The tip 122 of the surgical instrument 119 is illustrated on the right hand side of Fig. 5. The surgical instrument 119 may for example be an irrigation/aspiration instrument for irrigating or aspirating fluid at a desired target location, a photocoagulation instrument for applying optical energy at a desired distance from a target, a vitrectomy instrument for cutting and aspirating the vitreous humor or other fluidics at a desired distance from a target, a tissue manipulating instrument, which may need to be placed at the first interface of a tissue, or the like.

In some embodiments, a sensor or sensor component 121, such as an optical fibre, may be integrated in or attached to the surgical instrument 119. The sensor may comprise an optical fibre (as shown) coupled to an OCT sensor and configured to emit an optical beam 127 to capture cross-sectional images of the retina 210 and other structures within the eye. The optical fibre 121 may be recessed relative to the tip 122 of the surgical instrument 119. Since the OCT probe may be longitudinally forward facing, the sensor data may be indicative of the longitudinal distance D to a retinal surface structure laterally forward of the surgical instrument 119, which may be considered to be 'beneath' or 'ahead' of the surgical instrument 119. It will be appreciated that since the distance D may be larger than the distance of the tip 122 to the retinal surface structure, the distance D may be considered to underestimate the proximity of the tip 122 to the retinal surface structure. However, since the dimensions of the surgical instrument 119 and its relative position to the optical fibre 121 or other sensor are typically known, the processor subsystem may determine the distance between the retinal surface structure to any part of the surgical instrument 119, including its tip 122, using such known dimensions/relative positions.

It is noted that when the surgical instrument 119 is operated in the periphery of the eye, or in the vicinity of the posterior side of the lens in vitrectomy, the retina and/or the retinal surface structure may also be sideways proximate to the grasping device, and in some cases more proximate sideways than longitudinally. To account for such sideward proximity, the OCT probe may be a multi-directional probe with axial and side-viewing capability so as to be able to determine the distance to the retinal surface structure and/or retina also sideward to the tip 122 of the surgical instrument 119.

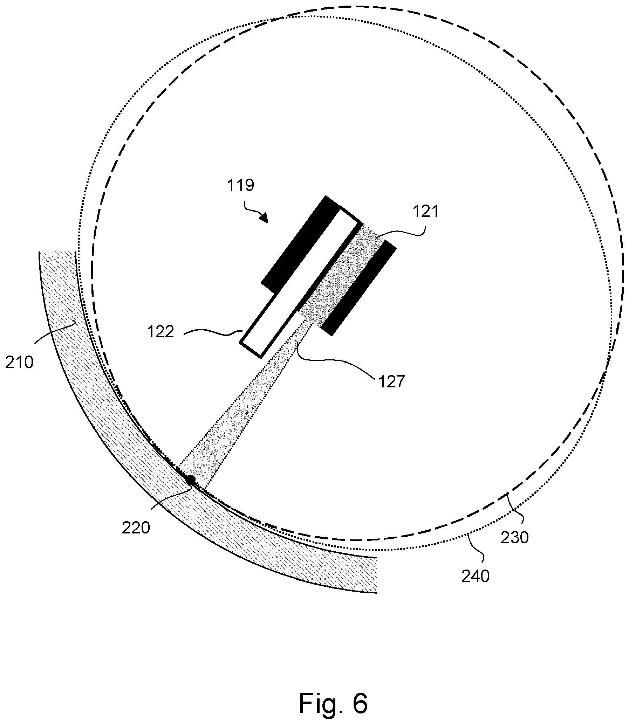

**Fig. 6** illustrates the processor subsystem of the surgical robotic system using at least a first model and a second model in the model-assisted control of the surgical instrument. Each model may be configured to model at least part of a surgical environment. During the intraocular procedure, the processor subsystem may access sensor data captured by a sensor as described in this specification, adapt at least one of said models to the sensor data to update said model to better model a currently captured part of the surgical environment, assess one or more switching criteria that define conditions for switching between the first model and the second model for the model-assisted control, and when the one or more switching criteria are met, switch between the first model and the second model for the model-assisted control.

In the specific example of Fig. 6, the processor subsystem is shown to use two global models 230, 240 which each model an interior of the eye, e.g., a shape of a curved plane representing the retinal surface. One of the models is shown to be a spherical model 230 which models the interior of the eye by a sphere while the other model is an elliptical model 240 which models the interior of the eye by an ellipsoid. In a specific example, each model may be adaptable to model the current surgical environment. Such adaptation may be performed based on sensor data so as to approximate the shape of the retina of a particular patient. For example, the sensor data may be obtained from an intraoperative instrument-integrated OCT probe as described in this specification. Specifically, from such sensor data, distance measurements may be obtained which may be indicative of the distance between the surgical instrument at its current pose and the retinal surface. It is noted that the current pose of the surgical instrument may be known to the processor subsystem as the processor subsystem may be tasked with controlling the pose. Thereby, the processor subsystem may convert the distance measurements to data points in a coordinate system which is linked to the patient. Such data points may also be referred to as measurement points as they may be derived from sensor measurements.

At the beginning of the surgical procedure, the spherical model 230 may be fitted to the data points obtained from the sensor data and the fitted spherical model 230 may be used in the model-assisted control. During the surgical procedure, the processor subsystem may switch to the elliptical model 240 for greater accuracy as more data points are collected. Before such switching, the elliptical model may already be adapted to the sensor data. For example, both the spherical model 230 and the elliptical model 240 may be concurrently adapted to the sensor data. However, this is not a limitation, in that when the elliptical model 240 is not in use in the model-assisted control, the processor subsystem may refrain from adapting the elliptical model 240 and only start such adaptation when the elliptical model is deemed suitable for use in the model-assisted control, or only when its suitability is to be evaluated.

The sphere fitting may be explained as follows. A sphere may be parameterized by its center point $(x_0, y_0, z_0)$ and its radius $r$. An online sphere fitting problem can be derived from a general sphere equation as

$$arg\ min_{(x_0, y_0, z_0, r)}\ \left\| \begin{pmatrix} x_i^2 \\ y_i^2 \\ z_i^2 \\ 1 \end{pmatrix} \cdot \begin{pmatrix} 2x_0 \\ 2y_0 \\ 2z_0 \\ r^2 - x_0^2 - y_0^2 - z_0^2 \end{pmatrix} - \left( x_i^2 + y_i^2 + z_i^2 \right) \right\|,$$

to obtain linear dependencies between a given measurement point $(x_i, y_i, z_i)$, and parameters $(x_0, y_0, z_0, r)$ to be optimized. Here, the term 'online fitting' may refer to the modelling of the surgical environment not being available at the beginning of the surgical procedure, but being acquired progressively by acquiring measurement points during the procedure and the model being continuously updated to better fit the acquired measurement points. To solve this online sphere fitting problem, a Kalman filter may be used or any other suitable optimization technique.

The ellipsoid fitting may be explained as follows. An ellipsoid may be parameterized by its center point $(x_0, y_0, z_0)$, semi-axes $(r_1, r_2, r_3)$ and orientation $(\alpha, \beta, \gamma)$. For example, the residual of the general quadratic equation

$$ax^2 + by^2 + cz^2 + 2fyz + 2gxz + 2hxy + 2px + 2qy + 2rz + d = 0$$

may be minimized to obtain linear dependencies between the given measurement point $(x_i, y_i, z_i)$ and the parameters $(a, b, c, f, g, h, p, q, r, d)$, and to avoid nonlinear optimization. An additional nonlinear constraint

$$4(ab + bc + ac - f^2 - g^2 - h^2) - (a + b + c)^2 > 0$$

may be used to specifically fit ellipsoids. The Kalman filter may be extended to deal with such nonlinear constraints. Another option is to use Cayley Transform Ellipsoid Fitting (CTEF) to find the best fit parameters.

As elucidated above, sphere fitting may be considered as a linear optimization without constraints. The optimization involved in sphere fitting may thus be relatively robust even when the data points are concentrated near a relatively small subset of the sphere. Ellipsoid fitting, on the other hand, may represent a difficult to solve optimization problem, especially when the data points are scarce, noisy and/or non-uniform. Accordingly, the processor subsystem may switch for the model-assisted control of the surgical instrument from the spherical model to the elliptical model only when one or more switching criteria are satisfied. Such switching criteria may for example comprise a model certainty which may be defined as the sum of the residuals of the measurement points with respect to the respective model. The model certainty criterion may favor the model that has a smaller sum of residuals. Another switching criterion may be the temporal variation of a respective model. For example, the temporal ellipsoid variation may be used as a criterion to switch from sphere to ellipsoid when the relative change of the global ellipsoid parameters, for example in relation to the ellipsoid axes, is below a certain percentage within a time period. For example, the criterion may trigger a transition to the elliptical model when the variation is less than 1% within a time period $s$. Exemplary values for $s$ may be 1, 2, 5, 10, 20, 30, 60 or 120 seconds. In another example, the time period $s$ may be dependent on the number of measurements, in that the relative change may be assessed after a number of measurements have been made. The processor subsystem may combine several switching criteria, for example using weighting and thresholding, majority selection, or by having specific switching criteria trigger specific switches to a specific model, etc.

While the above refers to a spherical model and an ellipsoid model, both models may also model different geometrical shapes, or in general, may represent any types of models which are configured to model at least part of the surgical environment, e.g., geometrically, biomechanically, or otherwise, and which differ by one or more characteristics. To determine when to switch between the models, the processor subsystem may access data which at least partially characterizes a current state of the surgical robotic system and/or the intraocular procedure, and the one or more switching criteria may be defined to determine a utility and/or suitability of a respective model in the current state by evaluating the utility and/or suitability of the characteristic of the respective model in the current state. By determining the current state of the surgical robotic system and/or the intraocular procedure, the processor subsystem may obtain a characterization of the present situation and compare the characterization of the present situation to the characteristics of the respective model to determine which model is most suitable for the present situation. For example, the processor subsystem may be configured to determine the current state and thus the present situation based on data which is indicative of the current pose of the surgical robotic system, recent actions performed with the surgical robotic system, surgical planning data defining a surgical plan for the surgical procedure, sensor data, etc.

The switching criteria may thus be defined to effect a switch in various specific situations. For example, a switching criterion may be defined to effect a switch from a first model to a second model when the scope of the first model is reached. For example, if the first model is a local model modelling a local part of the surgical environment, the switching criterion may be defined to effect a switch to a global model once the surgical instrument moves outside of the area which is modelled by the first model. Another example is that a switching criterion may be defined in relation to a presumed reliability of a model. The presumed reliability may for example be determined based on a confidence level outputted by a respective model, based on a comparison between a modelled quantity by the respective model and an observed quantity from the sensor data, based on a defined amount of sensor data having been used to adapt the model, and/or based on a temporal stability of a modelling by the respective model. Another example is that a switching criterion may be defined to effect the switch when a defined distance to an anatomical structure is reached. For example, in case the first model and the second model provide different levels of detail of an anatomical structure, the processor subsystem may switch to the higher-detailed model when the surgical instrument arrives in a vicinity of the anatomical structure, and switch to the lower-detailed model when the surgical instrument leaves the vicinity. Another example is that a switching criterion may be defined to effect the switch when a defined movement speed of the surgical instrument is exceeded. For example, in case the first model and the second model are different in terms of computational complexity to update and/or evaluate, the processor subsystem may switch to the more efficient model when the surgical instrument moves faster and switch to the less computationally efficient model, this being typically a more accurate and/or detailed model, when the surgical instrument slows down. Another example is that a switching criterion may be defined to effect the switch when a defined surgical phase is reached. The surgical phase may for example be estimated based on a pose of and/or actions performed with the surgical instrument or based on surgical planning data. Different models may have a different suitability for different surgical phases. Accordingly, the processor subsystem may switch to the most suitable model for each respective surgical phase. Another example is that a switching criterion may be defined to effect the switch when the sensor data is unavailable or unreliable. For example, the processor subsystem may switch to a model which is deemed to be reliable even if the sensor data is unavailable or unreliable. This model may also be regarded as a fallback model and may be a less complex model, for example a non-adaptive model.

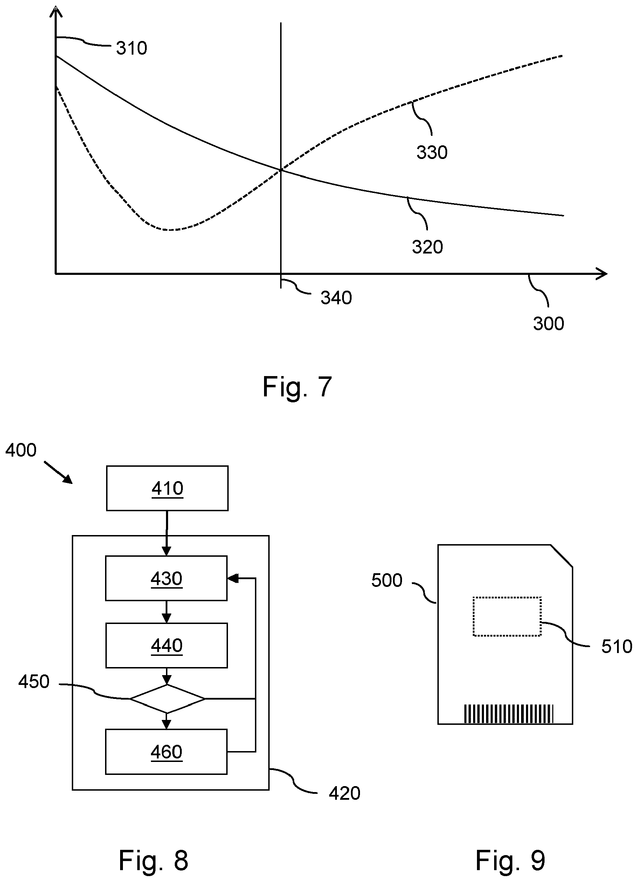

**Fig. 7** shows examples of utility functions 320, 330 for two models, with each utility function expressing a utility 310 of a respective model. Fig. 7 shows the utility on the vertical axis 310 over an exemplary period of time on the horizontal axis 300. In general, a utility function may represent a function which numerically expresses the utility of a respective model. In other words, a utility function may provide a numerical quantification of the utility as output. As input to the utility function, data may be used as indicated in the preceding paragraph or elsewhere in this specification, e.g., the confidence level of a model, the current pose of the surgical instrument, the current surgical phase, etc. The numerical output of the utility function may be used by the processor subsystem to determine when to switch models. The switching criteria may thus be defined in relation to the numerical output of the utility function, for example by comparing the numerical output to a reference. For example, if the numerical quantification of the utility of a respective model exceeds a certain threshold, e.g.,

the aforementioned reference, the processor subsystem may switch to the respective model. Fig. 7 shows an example in which the processor subsystem compares the utility of two models and switch between the models once the utility of one model surpasses that of the other model. Fig. 7 shows the switching by a vertical line 340. It will be appreciated that various other switching strategies are equally conceived. For example, the processor subsystem may be configured to switch earlier, e.g., in anticipation that the model will be more suited in the near future, or at a later moment, etc.

In general, the first model and the second model may differ in terms of complexity, for example in terms of their capability to model the surgical environment or in terms of a computational complexity of updating and/or evaluating said models. Examples of differences in complexity as well as other differences between the models include, but are not limited to, the number of parameters which define a respective model, the amount of sensor data which is needed to obtain a satisfactory accurate modelling of the surgical environment, the level of detail which a respective model is capable of modelling, the extent of the surgical environment, e.g., the spatial extent, which a respective model is capable of modelling, the computational complexity of evaluating a respective model, the computational complexity of updating a respective model to new sensor data, the average level of confidence of predictions provided by a respective model, the type of model, for example being a machine learning model and a non-machine learning model or different types of machine learning models.

In general, a respective model may be a geometric model, e.g., defining a geometry of the surgical environment, or a biomechanical model, e.g., defining a biological mechanism in the surgical environment, or a combined geometric and biomechanical model. One or both models may be adaptable models, in that they may be adapted based on sensor data to fit the surgical environment at hand. Examples of adaptable models include, but are not limited to, parameterized models of which one or more parameters may be adapted to better model the surgical environment, and machine learning models of which one or more model parameters and/or hyperparameters may be adapted, for example based on continuous learning, incremental learning, or online learning-based training techniques. A plurality of adaptable models may be adapted based on the same type of sensor data or based on different sensor data, e.g., obtained from different sensors. While various references are made to the switching between two models, it will be appreciated that any number of models may be used by the processor subsystem, e.g., three, four, five, or more. The processor subsystem may thus switch between a plurality of models. It will be further appreciated that in some embodiments, neither of the models may be adapted during the surgical procedure. It will be appreciated that such models may nevertheless be patient specific. For example, a respective model may be generated or adapted based on preoperative sensor data to obtain a patient-specific model.

It will be appreciated that while the above refers to the sensor data being indicative of distances and the distances being used to adapt a respective model, different types of models may be adapted to different types of sensor data. For example, a biomechanical model may be adapted based on output from a force sensor.

The switching between models may comprise a transitional phase. For example, when or after deciding to switch between models, the processor subsystem may gradually transition between the models, for example by temporarily combining the output of both models. The respective outputs may for example comprise simulated or predicted quantities, such as responses, interactions, boundaries, etc. Combining the outputs may for example comprise weighting the outputs, performing a majority selection amongst the outputs when more than two models are used, etc. Another form of transition may be obtained by temporarily combining the modelling by the models themselves, which may yield a hybrid model. In other examples, the switching between models may be a hard switch in that the switching may lack a transitional phase.

In general, the processor subsystem described in this specification may comprise one or more (micro)processors which execute appropriate software. The software implementing the functionality of the processor subsystem may have been downloaded and/or stored in a corresponding memory or memories, e.g., in volatile memory such as RAM or in non-volatile memory such as Flash. Alternatively, the processor subsystem may be implemented in the form of programmable logic, e.g., as a Field-Programmable Gate Array (FPGA). Any input and/or output interfaces may be implemented by respective hardware and/or software interfaces. In general, the processor subsystem, input interfaces, and/or output interfaces may each be implemented in the form of a circuit or circuitry. The processor subsystem may also be implemented in a distributed manner, e.g., involving different devices or apparatus.

**Fig. 8** shows a method 400 of controlling a surgical robotic system during a surgical procedure. The surgical robotic system may be of a type as described in the specification. The method 400 may comprise accessing 410 at least a first model and a second model, wherein each model is configured to model at least part of the surgical environment, and during the intraocular procedure 420, accessing 430 sensor data captured by a sensor, adapting 440 at least one of said models to the sensor data to update said model to better model a currently captured part of the surgical environment, assessing 450 one or more switching criteria that define conditions for switching between the first model and the second model for the model-assisted control, and when the one or more switching criteria are met, switching 460 between the first model and the second model for the model-assisted control. It will be appreciated that steps 430-460 may be performed repeatedly during the surgical procedure, for example to repeatedly assess whether the switching criteria are met or not. In some examples, the adapting 440 of a respective model to the sensor data may take place only when it is decided to switch to the respective model, or shortly before such switching. In other words, the adapting of a respective model may be omitted or skipped when the respective model is not yet in use, for example when the switching criteria indicate that the respective model is not suitable in the present situation.

It is noted that any of the methods described in this specification, for example in any of the claims, may be implemented on a computer as a computer implemented method, as dedicated hardware, or as a combination of both. Instructions for the computer, e.g., executable code, may be stored on a computer-readable medium 500 as for example shown in **Fig. 9,** e.g., in the form of a series 510 of machine-readable physical marks and/or as a series of elements having different electrical, e.g., magnetic, or optical properties or values. The executable code may be stored in a transitory or non-transitory manner. Examples of computer-readable mediums include memory devices, optical storage devices, integrated circuits, servers, online software, etc. Fig. 9 shows by way of example a memory card 500.

It should be noted that the above-mentioned embodiments illustrate rather than limit the invention, and that those skilled in the art will be able to design many alternative embodiments without departing from the scope of the appended claims.

In the claims, any reference signs placed between parentheses shall not be construed as limiting the claim. Use of the verb "comprise" and its conjugations does not exclude the presence of elements or stages other than those stated in a claim. The article "a" or "an" preceding an element does not exclude the presence of a plurality of such elements. Expressions such as "at least one of" when preceding a list or group of elements represent a selection of all or of any subset of elements from the list or group. For example, the expression, "at least one of A, B, and C" should be understood as including only A, only B, only C, both A and B, both A and C, both B and C, or all of A, B, and C. The invention may be implemented by means of hardware comprising several distinct elements, and by means of a suitably programmed computer. In the device claim enumerating several means, several of these means may be embodied by one and the same item of hardware. The mere fact that certain measures are recited in mutually different dependent claims does not indicate that a combination of these measures cannot be used to advantage.

**Claims**

1.   A surgical robotic system (100) for use in an intraocular procedure, comprising:

- an end effector for holding a surgical instrument (119);
- an actuator subsystem (60);
- a sensor interface to a sensor (30) configured to capture sensor data (32) of at least part of a surgical environment during the intraocular procedure;
- a processor subsystem (40) configured to:
- control the actuator subsystem (60) to control a pose of the surgical instrument (119) during the intraocular procedure; and
- in the control, use a model (230, 240) which models the surgical environment to establish a model-assisted control of the surgical instrument (119);

wherein the processor subsystem (40) is further configured to:

- access at least a first model (230) and a second model (240), wherein each model is configured to model at least part of the surgical environment;

and during the intraocular procedure:

- access the sensor data (32) captured by the sensor;
- adapt at least one of said models (230, 240) to the sensor data (32) to update said model to better model a currently captured part of the surgical environment;
- assess one or more switching criteria that define conditions for switching between the first model (230) and the second model (240) for the model-assisted control; and
- when the one or more switching criteria are met, switch between the first model (230) and the second model (240) for the model-assisted control.

2.   The surgical robotic system (100) according to claim 1, wherein the first model (230) and the second model (240) differ by at least one characteristic, wherein the processor subsystem (40) is configured to access data which at least partially characterizes a current state of the surgical robotic system and/or the intraocular procedure, and wherein the one or more switching criteria are defined to determine a utility and/or suitability of a respective model in the current state by evaluating the utility and/or suitability of the characteristic of the respective model in the current state.

3. The surgical robotic system (100) according to claim 2, wherein the one or more switching criteria are defined to compare the utility and/or suitability of both models to determine when to switch between the first model and the second model, or defined to compare the utility and/or suitability of a respective model to a reference.

4. The surgical robotic system (100) according to any one of claims 1 to 3, wherein the one or more switching criteria are defined to switch from the first model (230) to the second model (240):

   - when a scope of the first model is reached;
   - when the second model is deemed sufficiently reliable, for example based a confidence level outputted by the second model or based on a comparison between a modelled quantity by the second model and an observed quantity from the sensor data or based on a defined amount of sensor data having been used to adapt the second model or based on a temporal stability of a modelling by second model;
   - when a defined distance to an anatomical structure is reached, with the second model providing a different level of detail of the anatomical structure;
   - when a defined movement speed of the surgical instrument (119) is exceeded, with the second model being more computationally efficient to update and/or evaluate than the first model;
   - when a defined surgical phase is reached, with the second model being configured to be used in the defined surgical phase;
   - when the sensor data (32) is unavailable or unreliable, with the second model being a fallback model for such unavailability or unreliability of the sensor data.

5. The surgical robotic system (100) according to any one of claims 1 to 4, wherein the first model (230) and the second model (240) differ in terms of complexity, for example in terms of their capability to model the surgical environment or in terms of a computational complexity of updating and/or evaluating said models.

6. The surgical robotic system (100) according to any one of claims 1 to 5, wherein the first model (230) and the second model (240) differ in terms of:

   - a number of parameters which define a respective model;
   - an amount of sensor data (32) which is needed to obtain a satisfactory accurate modelling of the surgical environment;
   - a level of detail which a respective model is capable of modelling;
   - an extent of the surgical environment which a respective model is capable of modeling;
   - a computational complexity of evaluating a respective model;
   - a computational complexity of updating a respective model to new sensor data;
   - an average level of confidence of predictions provided by a respective model;
   - a type of model, for example a machine learning model and a non-machine learning model or different types of machine learning models.

7. The surgical robotic system (100) according to any one of claims 1 to 6, wherein one or each respective model (230, 240) is a geometric model and/or a biomechanical model of the surgical environment.

8. The surgical robotic system (100) according to any one of claims 1 to 7, wherein the processor subsystem (40) is configured to adapt both the first model (230) and the second model (240) based on sensor data (32) captured during the intraocular procedure.

9. The surgical robotic system (100) according to any one of claims 1 to 8, wherein the sensor interface is configured to, during the intraocular procedure, access first sensor data captured by a first sensor and second sensor data captured by a second sensor, wherein the processor subsystem (40) is configured to adapt the first model (230) based on the first sensor data and the second model (240) based on the second sensor data.

10. The surgical robotic system (100) according to any one of claims 1 to 9, wherein one or each respective model (230, 240) is:

   - a parameterized model, wherein the parameterized model is adapted to the sensor data by adapting one or more parameters of the parameterized model; or
   - a machine learning model, wherein the machine learning model is adapted to the sensor data by continuous, incremental, or online learning.

11. The surgical robotic system (100) according to any one of claims 1 to 10, wherein the first model is a global model of the eye and wherein the second model is a local model of a part of the eye.

12. The surgical robotic system (100) according to any one of claims 1 to 10, wherein the first model (230) is a spherical model of the eye and wherein the second model (240) is an ellipsoid model of the eye.

13. The surgical robotic system (100) according to any one of claims 1 to 12, wherein the sensor (30) comprises an OCT probe (121), for example integrated into or attached to the surgical instrument.

14. The surgical robotic system (100) according to any one of claims 1 to 13, wherein the processor subsystem (40) is configured to use a respective model (230, 240) in the control of the actuator subsystem (60) to:

- limit an adjustment of the pose of the surgical instrument by a human operator of the surgical robotic system;
- adjust the pose of the surgical instrument;
- perform an action with the surgical instrument;
- adjust an operating parameter of the surgical instrument; and/or
- provide sensory-perceptible feedback to the human operator.

15. A computer-implemented method (400) for controlling a surgical robotic system during use in an intraocular procedure, wherein the surgical robotic system comprises:

- an end effector for holding a surgical instrument;
- an actuator subsystem;
- a sensor interface to a sensor configured to capture sensor data of at least part of a surgical environment during the intraocular procedure;
wherein the method comprises:

- controlling the actuator subsystem to control a pose of the surgical instrument during the intraocular procedure;
- in the control, using a model which models the surgical environment to establish a model-assisted control of the surgical instrument;
wherein the method further comprises:

- accessing (410) at least a first model and a second model, wherein each model is configured to model at least part of the surgical environment;
and during the intraocular procedure (420):

- accessing (430) the sensor data captured by the sensor;
- adapting (440) at least one of said models to the sensor data to update said model to better model a currently captured part of the surgical environment;
- assessing (450) one or more switching criteria that define conditions for switching between the first model and the second model for the model-assisted control; and
- when the one or more switching criteria are met, switching (460) between the first model and the second model for the model-assisted control.

16. A transitory or non-transitory computer-readable medium (500) comprising data (510) representing a computer program, the computer program comprising instructions for causing a processor system to perform the method according to claim 15.

Fig. 1

Fig. 2

Fig. 3

Fig. 4

Fig. 5

Fig. 6

310

330

320

340

300

Fig. 7

400

410

430

440

450

460

420

Fig. 8

500

510

Fig. 9

Europäisches
Patentamt

European
Patent Office

Office européen
des brevets

## EUROPEAN SEARCH REPORT

Application Number

EP 24 16 0208

### DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| Y | WO 2019/222228 A1 (UNIV CALIFORNIA [US]) 21 November 2019 (2019-11-21) * paragraphs [0033], [0036], [0039] - [0045], [0083] - [0086], [0090], [0094], [0113]; figure 16 * | 1-16 | INV. A61B34/32 A61B17/28 A61B34/37 A61F9/007 |
| Y | US 2020/345431 A1 (PATTON DOUGLAS [US]) 5 November 2020 (2020-11-05) * paragraph [0070] * * paragraphs [0066], [0067], [0075], [0103] * | 1-16 | ADD. A61B17/00 A61B90/00 |
| A | DE 10 2020 106607 A1 (ZEISS CARL MEDITEC AG [DE]) 16 September 2021 (2021-09-16) * paragraphs [0058], [0060]; figures 1,2 * | 9 | |
| A | WO 2016/030336 A1 (UNIV EINDHOVEN TECH [NL]) 3 March 2016 (2016-03-03) * pages 14,15; figures 10,11 * | 11,12 | |

TECHNICAL FIELDS SEARCHED (IPC)

A61B
A61F

The present search report has been drawn up for all claims

| Place of search | Date of completion of the search | Examiner |
|---|---|---|
| The Hague | 7 August 2024 | Schmidt, Matthias |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## ANNEX TO THE EUROPEAN SEARCH REPORT
## ON EUROPEAN PATENT APPLICATION NO.

EP 24 16 0208

This annex lists the patent family members relating to the patent documents cited in the above-mentioned European search report.
The members are as contained in the European Patent Office EDP file on
The European Patent Office is in no way liable for these particulars which are merely given for the purpose of information.

07-08-2024

| Patent document cited in search report | Publication date | Patent family member(s) | | Publication date |
|---|---|---|---|---|
| WO 2019222228 A1 | 21-11-2019 | EP | 3793425 A1 | 24-03-2021 |
| | | US | 2021228292 A1 | 29-07-2021 |
| | | WO | 2019222228 A1 | 21-11-2019 |
| US 2020345431 A1 | 05-11-2020 | AU | 2020270386 A1 | 02-12-2021 |
| | | BR | 112021022049 A2 | 22-03-2022 |
| | | CA | 3138958 A1 | 12-11-2020 |
| | | CN | 114269302 A | 01-04-2022 |
| | | EP | 3962424 A1 | 09-03-2022 |
| | | JP | 2022530834 A | 01-07-2022 |
| | | KR | 20210156839 A | 27-12-2021 |
| | | TW | 202046341 A | 16-12-2020 |
| | | US | 2020345431 A1 | 05-11-2020 |
| | | US | 2024252256 A1 | 01-08-2024 |
| | | WO | 2020227026 A1 | 12-11-2020 |
| DE 102020106607 A1 | 16-09-2021 | DE | 102020106607 A1 | 16-09-2021 |
| | | US | 2021286996 A1 | 16-09-2021 |
| WO 2016030336 A1 | 03-03-2016 | CN | 106659539 A | 10-05-2017 |
| | | EP | 3185806 A1 | 05-07-2017 |
| | | EP | 4101412 A1 | 14-12-2022 |
| | | ES | 2922526 T3 | 16-09-2022 |
| | | US | 2017252113 A1 | 07-09-2017 |
| | | US | 2019282310 A1 | 19-09-2019 |
| | | US | 2021236219 A1 | 05-08-2021 |
| | | US | 2024180636 A1 | 06-06-2024 |
| | | WO | 2016030336 A1 | 03-03-2016 |

EPO FORM P0459

For more details about this annex : see Official Journal of the European Patent Office, No. 12/82

**EP 4 609 820 A1**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 10350014 B2